# EUROPEAN PATENT SPECIFICATION

(11) **EP 3 146 044 B1**
(45) Date of publication and mention of the grant of the patent: **10.07.2019**
(21) Application number: 15724572.1
(22) Date of filing: 14.05.2015
(51) Int. Cl.: C12N 11/02, C12N 11/08, C12P 7/64, C12N 9/20

(54) **PROCESS FOR IMMOBILIZATION OF A LIPASE**
VERFAHREN ZUR IMMOBILISIERUNG EINER LIPASE
PROCÉDÉ D'IMMOBILISATION D'UNE LIPASE

(30) Priority: 20.05.2014 EP 14169180
(43) Date of publication of application: 29.03.2017
(60) Divisional application: 19156967.2
(73) Proprietor: Bunge Loders Croklaan B.V., 1521 AZ Wormerveer (NL)
(72) Inventor: BHAGGAN, Krishnadath, NL-1521 AZ Wormerveer (NL); MA, Jun, NL-1521 AZ Wormerveer (NL); MIORINI, Chiara, I-31011 Asolo Treviso (IT); TARAN, Viktoria, 2512 AM Den Haag (NL)
(74) Representative: Potter Clarkson
(86) International application number: PCT/EP2015/060726
(87) International publication number: WO 2015/177042

(56) References cited:
- WO-A1-94/28118
- NEVENA, Z ET AL: "Immobilization of lipase from Candida rugosa on Sepabeads: the effect of lipase oxidation by periodates", BIOPROCESS BIOSYST ENG., vol. 34, 2011, pages 803-810, XP019938990, cited in the application
- PALOMO JOSE M ET AL: "Modulation of Mucor miehei lipase properties via directed immobilization on different hetero-functional epoxy resins. Hydrolytic resolution of (R,S)-2-butyroyl-2-phenylacetic acid.", JOURNAL OF MOLECULAR CATALYSIS B ENZYMATIC, vol. 21, no. 4-6, 17 February 2003 (2003-02-17), pages 201-210, XP002733870, ISSN: 1381-1177
- PALOMO, JOSE M ET AL.: "Interfacial adsorption of lipases on very hydrophobic support (octadecyl-Sepabeads): immobilization, hyperactivation and stabilization of the open form of lipases", JOURNAL OF MOLECULAR CATALYSIS B: ENZYMATIC, vol. 19, no. 20, 2002, pages 279-286, XP002733871, cited in the application
- R V S AMORIN ET AL: "chitosan from Syncephalastrum racemosum used as a film support for lipase immobilization", BIORESOURCE TECHNOLOGY, vol. 89, no. 1, 10 March 2003 (2003-03-10) , pages 35-39,
- HANDAYANI NURRAHMI ET AL: "Immobilization of Mucor miehei Lipase onto Macroporous Aminated Polyethersulfone Membrane for Enzymatic Reactions.", MEMBRANES 12 APR 2012, vol. 2, no. 2, 12 April 2012 (2012-04-12), pages 198-213,

## Description

### Technical Field

The present invention relates to process for immobilizing a lipase on a support having a functional amino group, a process for producing a triglyceride composition using said immobilized lipase and to the use of the lipase in transesterification reactions.

### Background Art

Lipases (E.C. 3.1.1.3), belonging to the group of enzymes, catalyse specifically ester bonds in tri-, di-, and mono-acylglycerols to glycerol and fatty acids. They further catalyse other reactions such as interesterifications, esterifications, acidolysis, alcoholysis and aminolysis. The high costs of lipases make enzymatic processes economically unattractive. Immobilization of the lipases is a way to increase the industrial susceptibility of lipases and allows recovery of the lipase protein. Lipases can be immobilized on different supports applying various ways of pretreatment of the support or the lipase.

Nevena et al. (NEVENA, Z. Immobilization of lipase from Candida rugosa on Sepabeads: the effect of lipase oxidation by periodates. Bioprocess Biosyst Eng. 2011, no.34, p.803-810.) describes the use of certain Sepabeads® having either amino functional groups or epoxy groups as suitable support for the immobilization of a non-specific lipase from Candida rugosa. Sepabeads® having amino functional groups needed activation with glutaraldehyde or sodium-periodate to show improved activity.

Palomo et al. (PALOMO, Jose M, et al. Interfacial adsorption of lipases on very hydrophobic support (octadecyl-Sepabeads): immobilization, hyperactivation and stabilization of the open form of lipases. Journal of Molecular Catalysis B: Enzymatic. 2002, vol.19, no.20, p.279-286.) tested the immobilization of various lipases on very hydrophobic support such as octadecyl-Sepabead®.

WO94/28118 describes a process for the immobilization of a lipase on hydrophobic support material (Accurel) using an aqueous solution of lipase enzyme and a non-ionic surfactant such as Tween 20.

Enzyme activity is vulnerable to immobilizations reagents such as glutaraldehyde or immobilization support. To secure enzyme stability and activity after immobilization of the enzyme, often non-lipase proteins are added such as hen egg album or bovin serum albumin. However, these animal proteins are known to cause allergic reactions.

There remains a need for a simplified immobilization method without additional activation of the support and the right choice of support which will retain lipase activity and stability such as thermo stability to enable the production of commercially relevant triglyceride compositions.

### Summary of invention

The objective of the present invention is to provide an immobilization process wherein pre-activation of the support could be avoided and if indeed a hydrophobic support such as Sepabeads® having octadecyl groups (EC-OD) are able to perform transesterification reaction to obtain products of commercial importance. Another aim of the present invention was to provide an immobilization process avoiding treatment with non-lipase protein such as animal derived albumin to secure activity and stability of the lipase to produce triglyceride compositions.

According to the present invention, there is provided a process for immobilizing a lipase on a support containing a functional amino group in the presence of a surface-active material in an aqueous solution, wherein the surface-active material is a non-ionic surfactant. The term functional amino group refers to an amino group which is engaged in interacting with or binding to the lipase and optionally, the support.

Further disclosed herein is a process for producing a triglyceride by enzymatic transesterification by using a lipase, which is immobilized on a support having a functional amino group.

Also disclosed herein is the use of the immobilized lipase for producing a triglyceride fat composition comprising at least 15% by weight OPO.

The support having a functional amino group can be any support having an amino group such as amino-epoxy, or alkyl amino having a carbon chain of C1-C24, preferably C2-C10. The support comprises a methacrylic polymer. Preferably the polymer forms a matrix.

A preferred support of the present invention contains a functional alkylamino group such as ethyl amino or hexyl amino.

The mechanism of action between the support and the lipase is either by ionic interaction or chemical binding, wherein the ionic interaction is preferred.

The surfactant can be formed from sugars, (both mono-di-and polysaccharides), polyols (e.g. sorbitan and sorbitol) or polyethylene glycols having molecular weight from 350 to 35000, such as PEG s 600, 1500, 4000. Very suitable non-ionic surfactants are polyoxyethylene sorbitan C8-C24 fatty acid esters, in particular those derived from lauric acid, such as Tween 20® or derived from oleic acid such as Tween 80®.

The surfactant concentration in the aqueous solution should be sufficient to ensure effective loading of the support by the enzyme. Very good results were obtained by applying an aqueous solution with a surfactant concentration of at least 0.01 wt%, preferably 0.01-10, most preferably 0.1-5wt.%.

An ideal amount of lipase in g to support in g is between 1-20 wt.% by weight, preferably 5-15% by weight.

The contact times applied can vary between wide ranges. Suitably, however, contact times between 1 and 72 hours are applied.

The aqueous lipase solution has preferable a concentration between 1 to 20 g/l.

Although the lipase enzyme can be any prior art lipase, a preference is expressed for a lipase which is selected from 1) 1,3-specific lipases from *Rhizomucor miehei, Rhizopus oryzae and Thermomyces lanuginosus 2)* lipases from *Penicillium camembertii* specific for the hydrolysis of partial glycerides , such as Amano G, and 3) lipases specific for the hydrolysis of esters or triglycerides, preferably a lipase from *Candida rugosa.* In particular preferred is a 1 ,3- specific lipase from *Rhizopus oryzae* such as Lipase D from Amano.

Immobilization of the lipase can be performed in many different ways. Suitably, the contact between support, lipase and/or surfactant is performed as a batch process, as a continuous process in a fixed bed, as a continuous process in a fluidized bed or in a continuously stirred tank, while the contacting is performed with a continuous motion of the lipase solution.

The immobilized lipase according to the invention can be applied in any enzymatic conversion process, such as hydrolysis of triglycerides, diglycerides or esters, but also the esterification or transesterification of fatty acids or diglycerides or triglycerides. These processes are also disclosed herein, with the prerequisite that an immobilized lipase according to our invention be used in the process.

Preferred processes for making triglyceride is the production of triglycerides compositions comprising symmetrical triglycerides of the general formula ABA, such as OPO or SOS, wherein O is oleic acid, P is palmitic acid and S is a saturated fatty acid selected from palmitic acid and stearic acid. A particular preferred triglyceride composition of the invention comprises at least 15% by weight OPO.

Triglyceride fats and oils are important commercial products and are used extensively in, for example, the food industry. Some triglycerides are nutritionally important and the triglyceride 1 ,3-dioleoyl-2-palmitoyl glyceride (OPO) is known to be an important component of human milk fat.

### Examples

The following non-limiting examples illustrate the invention and do not limit its scope in any way. In the examples and throughout this specification, all percentages, parts and ratios are by weight unless indicated otherwise.

### Example 1 : Various Sepabeads® with aqueous Lipase D preparation

Preparation of the lipase solutions: Seven lipase solutions were prepared according to Table 1. Sample N°7 was the control sample. All reagents were mixed at 150 rpm at room temperature between 3 to 24 hours and then centrifuged to receive the immobilized lipase as a pellet.

**Table 1**

| Sample, N° | Sepabeads® (functional group) | Amount of Sepabeads in g | Lipase in g |
|---|---|---|---|
| 1 | EC-HA (Hexylamino) | 1.5 | 0.12 in 70 ml |
| 2 | EC-OD (Octadecyl) | 1.5 | 0.12 in 70 ml |
| 3 | EC-BU (Butyl) | 1.5 | 0.12 in 70 ml |
| 4 | EC-HFA (Amino-Epoxy) | 1.5 | 0.12 in 70 ml |
| 5 | EC-EA (Ethylamino) | 1.5 | 0.12 in 70 ml |
| 6 | EC-EP (Epoxy) | 1.5 | 0.12 in 70 ml |
| 7 | No support | 0 | 0.18 in 75 ml (equal to 0.12 in 70 ml) |

### Example 2 (comparative)

The acidolysis reaction was performed at 60 °C with all seven lipase preparations using the following acidolysis assay:
1 g Immobilized enzyme (use the pellet after centrifugation)
35 g Palm oil stearin fraction (Feedstock)
49 g Oleic acid
0.126 g H₂O

Composition Feedstock to be found in Table 2.

**Table 2**

| | Feedstock |
|---|---|
| Carbon number | |
| C48 | 62.1 |
| C50 | 24.3 |
| C52 | 8.6 |
| C54 | 1.9 |
| C56 | 0.0 |

The carbon number was determined by GC according to AOCS Ce 5.86.

Table 3 provides the results of the various acidolysis reactions of feedstock after 24 h.

**Table 3**

| | HA | OD | BU | HFA | EA | EP | Control |
|---|---|---|---|---|---|---|---|
| Carbon number | | | | | | | |
| C48 | 59.9 | 60.1 | 60.0 | 57.4 | 60.3 | 60.1 | 60.3 |
| C50 | 25.4 | 25.3 | 25.3 | 26.8 | 25.1 | 25.3 | 25.2 |
| C52 | 9.2 | 9.2 | 9.2 | 10.2 | 9.1 | 9.2 | 9.1 |
| C54 | 2.1 | 2.1 | 2.2 | 2.5 | 2.1 | 2.1 | 2.1 |
| C56 | 0.2 | 0.1 | 0.2 | 0.2 | 0.2 | 0.2 | 0.2 |

After 24 h nearly no product OPO or OOP (C52) was produced for all lipase preparations

### Example 3

70 ml of the lipase preparation of Example 1 was mixed with 2.4 g hen egg albumin, 0.65 g Tween 20® and 1.5 g of the respective supports. The acidolysis reaction was performed according to example 2.

Table 4 shows the results after acidolysis (24 hours) by using various sepabeads with aqueous lipase D solution in the presence of hen egg albumin and TWEEN 20.

**Table 4**

| | HA | OD | BU | HFA | EA | EP |
|---|---|---|---|---|---|---|
| Carbon number | | | | | | |
| C48 | 7.2 | 9.5 | 61.2 | 6.9 | 7.2 | 61.4 |
| C50 | 29.3 | 31.3 | 24.8 | 29.0 | 25.0 | 24.7 |
| C52 | 41.5 | 41.5 | 8.8 | 42.8 | 42.3 | 8.8 |
| C54 | 20.4 | 16.9 | 2.0 | 20.4 | 20.7 | 1.9 |
| C56 | 0.5 | 0.4 | 0.0 | 0.5 | 0.4 | 0.0 |

### Example 4

70 ml of the lipase preparation of Example 1 was mixed with 250 mg PEG 1500, 0.65 g Tween 20® and 1.5 g of the respective supports. The acidolysis reaction was performed according to example 2. As comparison immobilization on polypropylene (Accurel) under same reaction conditions was performed.

Table 5 shows the results after acidolysis (24 hours) by using various sepabeads with aqueous lipase D solution in the presence of PEG 1500 and Tween 20®.

**Table 5**

| | HA | OD | HFA | EA | Accurel |
|---|---|---|---|---|---|
| Carbon number | | | | | |
| C48 | 6.7 | 61.9 | 6.8 | 6.8 | 58.2 |
| C50 | 27.3 | 24.4 | 27.6 | 27.3 | 25.2 |
| C52 | 42.4 | 8.5 | 42.5 | 42.4 | 10.0 |
| C54 | 22.5 | 1.9 | 22.0 | 22.4 | 2.3 |
| C56 | 0.2 | 0.0 | 0.5 | 0.4 | 4.1 |

### Example 5

Multiple usage of Lipase D immobilized on support EC-HA.

70 ml of the lipase preparation of Example 1 was mixed with 30 mg PEG 600, 0.65 g Tween 20® and 1.5 g of support EC-HA. The acidolysis reaction was performed according to example 2. After 3.5 hours the acidolysis reaction was stopped and the immobilized lipase separated from the reaction mixture by filtration. The immobilized lipase is collected and used for the second run of the acidolysis assay . These runs were repeated eight times. At each run a sample (∼ 2 ml) at time 3.5 hours were taken for carbon number analysis.

Table 6 shows the results after acidolysis by reusing the immobilized lipase D on EC-HA support in subsequent 8 runs

**Table 6**

| | Run 1 | Run 2 | Run 3 | Run 4 | Run 5 | Run 6 | Run 7 | Run 8 |
|---|---|---|---|---|---|---|---|---|
| C46 | 0.8 | 1.2 | 1.3 | 1.5 | 1.4 | 1.5 | 1.5 | 1.6 |
| C48 | 11.2 | 19.6 | 22.7 | 25.3 | 24.7 | 26 | 27.7 | 28.5 |
| C50 | 34.1 | 35.9 | 36.4 | 35.9 | 35.8 | 35.6 | 35.3 | 35.2 |
| C52 | 42.4 | 34.2 | 31.3 | 29.4 | 30.1 | 29.1 | 28 | 27.4 |
| C54 | 11.2 | 9.1 | 8.2 | 7.8 | 8 | 7.8 | 7.6 | 7.3 |

### Example 6

Lipase D solution (0.9 g/77 ml) was mixed with various Tween in amounts provided in Table 7 and stirred for 15 min. To each of the preparations 1,5 g of Sepabead EC-HA was added and the mixture was stirred for 24 hours. Then immobilized enzyme was filtered off and tested in the acidolysis reaction as described in example 2. Table 7 shows the results of 5 different Tween's after acidolysis after 3.5 h.

**Table 7**

| Carbonnumber | Tween 20 | Tween 40 | Tween 60 | Tween 80 | Tween 85 |
|---|---|---|---|---|---|
| Amount in g | 0.650 | 0.676 | 0.693 | 0.694 | 0.974 |
| C46 | 1.5 | 2.14 | 2.79 | 1.34 | 2.4 |
| C48 | 16.6 | 41.19 | 54.37 | 18.02 | 46.5 |
| C50 | 34.2 | 31.4 | 27.49 | 33.26 | 30.5 |
| C52 | 36.9 | 18.73 | 11.85 | 36.11 | 15.8 |
| C54 | 10.6 | 5.78 | 3.48 | 10.77 | 4.5 |
| C56 | 0.3 | 0.53 | 0 | 0.38 | 0.3 |

### Example 7

Example 6 was repeated with Tween 80® with the difference that the premixing of the lipase solution with Tween 80® was skipped. Lipase solution, Tween 80® and support material were put together and the mixture was stirred for 24 hours. Then immobilized lipase was filtered off and tested in the acidolysis reaction as described in example 2. Table 8 shows the results after acidolysis after 3.5 h.

**Table 8**

| Carbonnumber | With premixing | No premixing |
|---|---|---|
| C46 | 1.3 | 1.51 |
| C48 | 13.8 | 16.38 |
| C50 | 33 | 32.32 |
| C52 | 40.2 | 38.34 |
| C54 | 11.4 | 11.14 |
| C56 | 0.3 | 0.3 |

The results demonstrate that premixing of Tween 80® with lipase solution is not required.

## Claims

1. A process for immobilizing a lipase on a support containing a functional amino group, which comprises contacting the lipase with said support in the presence of a surface-active material in an aqueous solution, wherein the surface-active material is a non-ionic surfactant.

2. A process according to claim 1, wherein the lipase is a 1 ,3 specific lipase.

3. A process according to claim 1 or 2, wherein the lipase is derived from *Rhizopus oryzae.*

4. A process according to any of the preceding claims, wherein the functional amino group is a functional alkylamino group having C1- C22 carbon atoms.

5. A process according to any of the preceding claims, wherein the surface-active material is selected from the group consisting of polyethyleneglycols, methoxy polyethyleneglycols, polysorbates and mixtures thereof.

6. A process according to any of the preceding claims, wherein the aqueous solution has a concentration of surface-active material of at least 0.01 wt%.

## Patentansprüche

1. Verfahren zur Immobilisierung einer Lipase auf einem Träger, die eine funktionelle Aminogruppe enthält, welche das Inkontaktbringen der Lipase mit dem Träger in Gegenwart eines oberflächenaktiven Materials in einer wässrigen Lösung umfasst, wobei das oberflächenaktive Material ein nicht-ionisches Tensid ist.

2. Verfahren nach Anspruch 1, wobei die Lipase eine 1,3-spezifische Lipase ist.

3. Verfahren nach Anspruch 1 oder 2, wobei die Lipase von *Rhizopus oryzae* stammt.

4. Verfahren nach einem der vorhergehenden Ansprüche, wobei die funktionelle Aminogruppe eine funktionelle Alkylaminogruppe mit C1-C22-Kohlenstoffatomen ist.

5. Verfahren nach einem der vorhergehenden Ansprüche, wobei das oberflächenaktive Material aus der Gruppe bestehend aus Polyethylenglykolen, Methoxypolyethylenglykolen, Polysorbaten und Mischungen davon ausgewählt ist.

6. Verfahren nach einem der vorhergehenden Ansprüche, wobei die wässrige Lösung eine Konzentration an oberflächenaktivem Material von mindestens 0,01 Gew.-% aufweist.

## Revendications

1. Procédé d'immobilisation d'une lipase sur un support contenant un groupe aminé fonctionnel, comprenant la mise en contact de la lipase avec ledit support en présence d'un matériau tensio-actif dans une solution aqueuse, dans laquelle la matière tensio-active est un tensioactif non ionique.

2. Procédé selon la revendication 1, dans lequel la lipase est une lipase spécifique 1,3.

3. Procédé selon la revendication 1 ou 2, dans lequel la lipase est dérivée du *Rhizopus oryzae.*

4. Procédé selon l'une quelconque des revendications précédentes, dans lequel le groupe aminé fonctionnel est un groupe alkylamino fonctionnel ayant des atomes de carbone C1-C22.

5. Procédé selon l'une quelconque des revendications précédentes, dans lequel le matériau tensio-actif est choisi dans le groupe constitué par les polyéthylèneglycols, les methoxy-polyéthylèneglycols, les polysorbates et leurs mélanges.

6. Procédé selon l'une quelconque des revendications précédentes, dans lequel la solution aqueuse présente une concentration de matériau tensio-actif avec au moins 0,01 % en poids.
